# EUROPEAN PATENT SPECIFICATION

(11) **EP 1 804 800 B1**
(45) Date of publication and mention of the grant of the patent: **22.12.2010**
(21) Application number: 05793868.0
(22) Date of filing: 17.10.2005
(51) Int. Cl.: A61K 31/506, A61P 25/28, A61P 35/00

(54) **USE OF 4-(4-METHYLPIPERAZIN-1-YLMETHYL)-N-4-METHYL-3-(4-(PYRIDIN-3-YL)PYRIMIDIN-2-YLAMINO)PHENYL¨-BENZAMIDE TO INHIBIT THE TYROSINE KINASE RECEPTOR C-FMS**
VERWENDUNG VON 4-(4-METHYLPIPERAZIN-1-YLMETHYL)-N-4-METHYL-3-(4-(PYRIDIN-3-YL)PYRIMIDIN-2-YLAMINO)PHENYL-BENZAMID ZUR HEMMUNG DES TYROSINKINASEREZEPTORS C-FMS
INHIBITION DU RÉCEPTEUR À TYROSINE KINASE C-FMS À L AIDE DU 4-(4-MÉTHYLPIPÉRAZIN-1-YLMÉTHYL)-N-4-MÉTHYL-3-(4-(PYRIDIN-3-YL)PYRIMIDIN-2-YLAMINO)PHÉNYL-BENZAMIDE

(30) Priority: 18.10.2004 US 619744 P
(43) Date of publication of application: 11.07.2007
(73) Proprietor: MEDVET SCIENCE PTY. LTD., Stepney, South Australia 5069 (AU)
(72) Inventor: DEWAR, Andrea, Louis, Kensington, S.A. 5068 (AU); HUGHES, Timothy, Peter, Kensington Gardens, S.A. 5068 (AU); LYONS, Alan, Bruce, Glengowrie, S.A. 5044 (AU)
(74) Representative: Roth, Peter Richard
(86) International application number: PCT/AU2005/001602
(87) International publication number: WO 2006/042362

(56) References cited:
- WO-A-2004/026930
- US-A- 4 837 237
- DEWAR A ET AL: "Imatinib inhibits C-FMS induced development and function of human monocytes in vitro" EXPERIMENTAL HEMATOLOGY (NEW YORK), vol. 32, no. 7, Suppl. 1, July 2004 (2004-07), page 87, XP009093621 & 33RD ANNUAL MEETING OF THE INTERNATIONAL-SOCIETY-FOR-EXPERIMENTAL-HEM ATOLOGY; NEW ORLEANS, LA, USA; JULY 17 -20, 2004 ISSN: 0301-472X
- MONTELLA LILIANA ET AL: "Imatinib mesylate for cerebral Langerhans'-cell histiocytosis." THE NEW ENGLAND JOURNAL OF MEDICINE 2 SEP 2004, vol. 351, no. 10, 2 September 2004 (2004-09-02), pages 1034-1035, XP002462063 ISSN: 1533-4406
- BAIOCCHI G ET AL: "EXPRESSION OF THE MACROPHAGE COLONY-STIMULATING FACTOR AND ITS RECEPTOR IN GYNECOLOGIC MALIGNANCIES" CANCER, vol. 67, no. 4, 1991, pages 990-996, XP002462103 ISSN: 0008-543X
- SLOMOVITZ B M ET AL: "Expression of imatinib mesylate-targeted kinases in endometrial carcinoma" GYNECOLOGIC ONCOLOGY, ACADEMIC PRESS, LONDON, GB, vol. 95, no. 1, October 2004 (2004-10), pages 32-36, XP004571347 ISSN: 0090-8258
- DEWAR ANDREA L ET AL: "Inhibition of c-fms by imatinib: expanding the spectrum of treatment." CELL CYCLE (GEORGETOWN, TEX.) JUL 2005, vol. 4, no. 7, July 2005 (2005-07), pages 851-853, XP009093618 ISSN: 1551-4005
- DEWAR A.L.: 'Macrophage colony-stimulating factor receptor c-fms is a novel target of imatinib' BLOOD vol. 105, no. 8, 15 April 2005, pages 3127 - 3132, XP008092299
- NETZER W.J. ET AL: 'Gleevec inhibits beta-amyloid production but not Notch cleavage' PROCEEDINGS OF THE NATIONAL ACADEMY OF SCIENCES vol. 100, no. 21, 2003, pages 12444 - 12449, XP008095037
- GLEEVECR CONSUMER INFORMATION, [Online] 19 December 2005, XP008097707 Retrieved from the Internet: <URL:http://web.archive.org/web/20020612153 200/http://www.fda.gov/cder/consumerinfo/dr uginfo/gleevec.htm>
- OKUDA K. ET AL: 'ARG tyrosine kinase activity is inhibited by STI571' BLOOD vol. 97, no. 8, 15 April 2001, pages 2440 - 2448, XP008092298
- BUCHDUNGER E.: 'Abl Protein-Tyrosine Kinase Inhibitor STI571 Inhibits In Vitro Signal Transduction Mediated by c-Kit and Platelet-Derived Growth Factor Receptors' THE JOURNAL OF PHARMACOLOGY AND EXPERIMENTAL THERAPEUTICS vol. 295, no. 1, 2000, pages 139 - 145, XP002359777
- EKLUND K. AND JOENSUU H.: 'Treatment of rheumatoid arthritis with imatinib mesylate: clinical improvement in three refractory cases' ANNALS OF MEDICINE vol. 35, no. 5, 2003, pages 362 - 367, XP009022024
- ROUSSIDIS A.E. ET AL: 'STI571 as a Potent Inhibitor of Growth and Invasiveness of Human Epithelial Breast Cancer Cells' ANTICANCER RESEARCH vol. 24, no. 3A, 2004, pages 1445 - 1448, XP009093570
- RAMADORI G. ET AL: 'Successful treatment of hepatocellular carcinoma with the tyrosine kinase inhibitor imatinib in a patient with liver cirrhosis' ANTI-CANCER DRUGS vol. 15, no. 4, 2004, pages 405 - 409, XP009093571
- MIYACHI K. ET AL: 'Efficacy of imatinib mesylate (STI571) treatment for a patient with rheumatoid arthritis developing chronic myelogenous leukemia' CLIN. RHEUMATOL. vol. 22, no. 4/05, 2003, pages 329 - 332, XP008046395
- ALVAREZ A.R. ET AL: 'Activation of the neuronal c-Abl tyrosine kinase by amyloid-beta-peptide and reactive oxygen species' NEUROBIOLOGY OF DISEASE vol. 17, 2004, pages 326 - 336, XP004592571
- KADOWAKI T. AND KUBOTA N.: 'Protective Role of Imatinib in Atherosclerosis' ARTERIOSCLER. THROMB. VASC. BIOL. vol. 24, no. 5, 2004, pages 801 - 803, XP008092295
- LASSILA M. ET AL: 'Imatinib Attenuates Diabetes-Associated Atherosclerosis' ARTERIOSCLER. THROMB. VASC. BIOL. vol. 24, no. 5, May 2004, pages 935 - 942, XP008037837
- DATABASE MEDLINE [Online] DEWAR A.L. ET AL: 'Imatinib inhibits the in vitro development of the monocyte/macrophage lineage from normal human bone marrow progenitors', XP008093102 Database accession no. 2003445226 & LEUKEMIA vol. 17, no. 9, September 2003, pages 1713 - 1721

## Description

The present invention relates to the use of 4-(4-methylpiperazin-1-ylmethyl)-N-[4-methyl-3-(4-(pyridin-3-yl)pyrimidin-2-ylamino)phenyl]-benzamide (Compound I) or a pharmaceutically acceptable salt thereof for the manufacture of a medicament for the treatment of c-fms-associated diseases selected from the group consisting of choriocarcinoma, embryonal carcinoma, brain microglial tumors, sarcoidosis, microglial cell involvement in normal and variant Creutzfeld-Jacob disease, or amyotrophic lateral sclerosis.

Macrophage-colony-stimulating factor (M-CSF or CSF-1), initially described as a growth factor of the mononuclear phagocytic lineage, also participates in immunological and inflammatory reactions, bone metabolism and pregnancy. The biological activities of M-CSF are mediated by a tyrosine kinase receptor c-fins. C-fms is a ligand inducible protein tyrosine kinase and belongs to the receptor subfamily III. The c-fins proto-oncogene encodes the only known receptor for the macrophage colony-stimulating factor-1 (CSF-1). It consist of a single transmembrane domain which separates the extracellular part, i.e. the ligand binding domain containing five immunoglobulin repeats from the intracellular -tyrosine kinase domain composed of two parts flanking a non-catalytic insertion sequence, the kinase insert. The M-CSF or CSF-1/tysorine kinase receptor.c-fms pair has essential physiological functions in monocyte and macrophage differentiation, embryogenic implantation, placenta development, and lactogenic differentiation of the human breast. The human mRNA for c-fms proto-oncogene is X03663 as accessible in ENTREZ www.ncbi.nlm.nih.gov. and see Coussens L et al., Nature 1986, 320(6059) 277-280.

Abnormal over-expression of M-CSF and c-fms, mRNA and proteins, is detected in primary tumors of epithelial origin. Abnormally high expression of the tyrosine kinase receptor c-fins has been associated with aggressive behaviour in a variety of malignancies, including breast, prostate, ovarian and endometrial cancers.

4-(4-methylpiperazin-1-ylmethyl)-N-[4-methyl-3-(4-(pyridin-3-yl)pyrimidin-2-ylamino)phenyl]-benzamide, referred herein below as, Compound I, is a protein kinase inhibitor showing high efficacy in the treatment of chronic myeloid leukemia, abbreviated as CML and gastrointestinal stromal tumors, abbreviated as GIST. Compound I targets the CML-specific tyrosine kinase bcr-abl but is also a potent inhibitor of the platelet derived growth factor receptor (PDGF-R), the stem cell factor (c-kit), c-abl and abl-related gene (ARG). In contrast to the tyrosine kinase receptors c-kit and PDGFRbeta, phosphorylation of the M-CSF receptor, c-fms, was reported to be unaffected by Compound I. Compound I has never been described as being useful for the inhibition of c-fms or for the manufacture of a medicament for the treatment of c-fms-associated diseases.

Compound I is 4-(4-methylpiperazin-1-ylmethyl)-N-[4-methyl-3-(4-(pyridin-3-yl)pyrimidin-2-ylamino)phenyl]-benzamide having the following formula

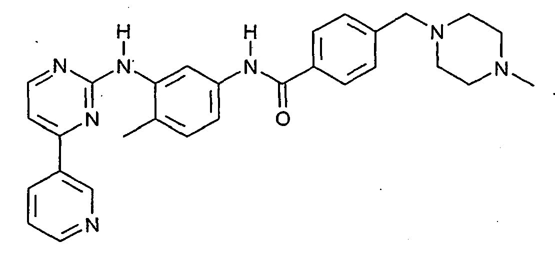

The preparation of the 4-(4-methylpiperazin-1-ylmethyl)-N-[4-methyl-3-(4-(pyridin-3-yl)pyrimidin-2-ylamino)phenyl]-benzamide is described in the EP-A-0 564 409.

Pharmaceutically acceptable salts of Compound I are pharmaceutically acceptable acid addition salts, for example, with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid.

The monomethanesulfonic acid addition salt of Compound I, hereinafter referred as "Salt I", and crystal forms thereof, e.g. the alpha-crystal form and the beta crystal form, are described e.g. in PCT patent application WO99/03854 published on January 28, 1999 and in the European patent No. 998 473.

All the WO (number) references are meant to refer to the WIPO publications of the PCT patent applications of the corresponding references.

It has now surprisingly been found that Compound I or a pharmaceutically acceptable salt thereof, e.g. Salt I, is capable of inhibiting the tyrosine kinase receptor c-fms that belongs to the receptor subfamily III and which is involved in the proliferation of an M-CSF dependent cell line.

The present invention relates to the use of Compound I or a pharmaceutically acceptable salt thereof, e.g. Salt I, for the preparation of a medicament for the treatment of c-fms-associated diseases, c-fins-associated neoplastic diseases and c-fms-associated non-neoplastic diseases selected from the group consisting of choriocarcinoma, embryonal carcinoma, brain microglial tumors, sarcoidosis, microglial cell involvement in normal and variant Creutzfeld-Jacob disease, or amyotrophic lateral sclerosis.

The present invention relates to the use of Compound I or a pharmaceutically acceptable salt thereof, e.g. Salt I, for the preparation of a medicament for the treatment of c-fins-associated cancers, e.g. c-fins-associated ovarian cancer, e.g. c-fms-associated ovarian serous carcinomas or c-fms-associated advanced epithelial ovarian carcinomas.

By c-fms-associated disease is meant a disease in which c-fms is involved, especially in which the c-fms protein or mRNA or both is over-expressed in comparison the patient not having a c-fms related disease. The present definition also encompass the cases where the level of the ligand of the c-fms receptor is overexpressed, and the case where the c-fms receptor is constitutively activated.

By c-fms-associated neoplastic diseases is meant the following diseases in which c-fms is involved: choriocarcinoma, embryonal carcinoma, brain microglial tumors.

By c-fms-associated non-neoplastic diseases is meant sarcoidosis, microglial cell involvement in normal and variant Creutzfeld-Jacob disease, and amyotrophic lateral sclerosis.

One embodiment of the invention relates to the use of Compound I or a pharmaceutically acceptable salt thereof, for the manufacture of a medicament to treat a c-fms-associated disease selected from the group consisting of choriocarcinoma, embryonal carcinoma, brain microglial tumors, sarcoidosis, microglial cell involvement in normal and variant Creutzfeld-Jacob disease, or amyotrophic lateral sclerosis.

According to one embodiment of the invention, Compound I or a pharmaceutical acceptable salt thereof, e.g. Salt I, is administered to the patient. The dosages are expressed as the dose of Compound I free base administered, e.g. for a 100 mg dose, 119.5 mg of Salt I is administered corresponding to 100 mg of Compound I free base. For example, a dose of 400 mg of Compound I has to be understood as 478 mg Salt I being administered corresponding to 400 mg of Compound I free base.

Depending on species, age, individual condition, mode of administration, and the clinical picture in question, effective doses of Compound I, for example, daily doses of about 100-1000 mg, e.g. 200 to 800 mg, e.g. 200-600 mg, e.g. 400 mg of Compound I, are administered to warm-blooded animals of about 70 kg bodyweight. For adult patients harbouring a c-fms mediated or c-fms-associated disease, a starting dose of 200- 400 mg of Compound I daily can be recommended. For patients with an inadequate response after an assessment of response to therapy with 400 mg of Compound I daily, dose escalation can be safely considered and patients may be treated as long as they benefit from treatment and in the absence of limiting toxicities. In this way, for example, one of skill in the art may determine an effective dose of Compound I or a pharmaceutically acceptable salt thereof to be administered to the patient.

According to the present invention, Compound I mesylate (Salt I), e.g. in the alpha crystal form, in the beta crystal form, or mixture thereof, is administered to the patient in need of a treatment of a c-fms-associated disease.

### Example I:Compound I or a pharmaceutically acceptable salt thereof inhibits the tyrosine kinase activity of the macrophage colony stimulating factor receptor c-fms at clinically relevant concentrations.

### Materials and Methods

*Isolation of Bone Marrow Mononuclear Cells (BMMNC).* Normal bone marrow (BM) is aspirated from the posterior iliac crest of healthy volunteers following informed consent. Low-density bone marrow mononuclear cells are collected by centrifugation over Ficoll-Hypaque (Lymphoprep, 1.077 g/dL; Nycomed Pharma) at 400g for 30 min.

*Isolation of CD34⁺ Cells.* CD34⁺ progenitor cells (>90% pure) are isolated from BMMNC using a MACS^{®} CD34⁺ progenitor cells selection isolation kit (Miltenyi Biotech), according to the manufacturers instructions.

*Haemopoietic Colony Assays.* BMMNC or CD34⁺ cells are assayed for colony formation in semisolid agar, using a modification of Johnson G.R. 1980. J Cell Physiol 103:371-383*.* Briefly, 5.0x10⁴ BMMNC or 7.5x10³ CD34⁺ cells are plated per 35mm cell culture dish (Falcon), in 1.0mL of IMDM (JRH Biosciences) supplemented with 0.33% agar (Bacto^{™} Agar, Difco), 25% FCS, and 2mM L-glutamine. Colony growth is stimulated by the addition of 4 growth factors ((4HGF) IL-3, IL-6, G-CSF, GM-CSF, each at a final concentration of 10 ng/mL) (Peprotech), 5 growth factors ((5HGF) IL-3, IL-6, G-CSF, GM-CSF, Stem cell factor (SCF), each at a final concentration of 10 ng/mL) (Peprotech), M-CSF (25 ng/mL) or GM-CSF (10 ng/mL) (Peprotech). Compound I (0.3 µM to 30 µM), anti-c-fins antibodies (2-4A5, Santa Cruz Biotechnology Inc) (1 µg/mL), or anti-c-kit antibodies (Sigma) (1 µg/mL) are also added to colony cultures. Cultures are incubated in a humidified chamber at 37°C + 5% CO₂ for a period of 2 weeks, after which time they are fixed in 3% glutaraldehyde. Fixed cultures are sequentially stained for naphthol acetate esterase, e.g. as described in Lojda, Z. *et a.l,* 1979. Enzyme histochemistry: a laboratory manual, Springer-Verlag, Berlin and chloroacetate esterase, see e.g. Kubota K. et al., 1980, Exp. Hematol. 8:339-44, then stained with luxol fast blue dye (BDH), to identify monocyte/macrophage, neutrophil and eosinophil colonies respectively. Colonies are scored according to standard criteria (>50 cells).

*M-CSF ELISA.* Monocytes are isolated from buffy coats from normal donors, as previously described, e.g. in Dewar A et al., 2003, Leukemia 17:1713-21. Monocyte cultures (1x10⁵/mL) are established in 24 well plates in serum deprived medium (SDM; IMDM/1% BSA supplemented with 2mM L-glutamine, 200 µg/mL transferrin, 10 µg/mL insulin (Actrapid^{®}, Novo Nordisk), 10⁻⁴M β-mercaptoethanol, 50 µg/mL low density lipoproteins (Sigma)) and stimulated with 20 ng/mL GM-CSF. Supernatants are harvested at 24 h intervals for 5 days, and analysed for M-CSF using an R&D Systems DuoSet^{®} ELISA development system according to the manufacturers instructions.

*Transduction of c-Fms into FDC-P1 Cells.* Stable Psi-2 virus-producing cell lines transfected with MSW/IRES/GFP/cfms (kindly provided by M. Roussel, St Jude Children's Research Hospital) are produced by Fugene (Roche) transfection, and sorted on a FACStar^{PLUS} flow cytometer (Becton Dickinson), collecting cells that expressed green fluorescence protein (GFP).. These cells are used to infect FDC-P1 cells by co-cultivation, and FDC-P1 cells expressing c-fms protein (FDC-cfins) are selected in DMEM supplemented with 10% FCS, 200 mM L-glutamine and 60 ng/mL rhM-CSF. *Proliferation Assays.* FDC-cfms cells are resuspended at 5.0x10⁴/mL in DMEM containing 10% FCS, and stimulated with murine IL-3 (1:2000) (kindly provided by Dr S. Read, IMVS) or rhM-CSF (60ng/mL) (Peprotech). Compound I is added to a final concentration of 0.5µM-5.0µM, in triplicate, and cells harvested at 12, 24 and 48 h time points. Cells are fixed in a known volume, and a fixed volume of known density Flow-Check^{™} Fluorospheres (Beckman Coulter) added. Cell number is determined using a Coulter XL-MCS analytical flow cytometer, using analysis based on gates on FS v SS plots that corresponded to beads/cells.

*Cell Lysates.* FDC-c-fms are incubated for 1h in serum free medium (DMEM, JRH Biosciences) at 37°C, +/- Compound I. Following starvation, cells are resuspended at 1.5x10⁷/mL in DMEM +/Compound I, stimulated with 60 ng/mL rhM-CSF for 2 min at 37°C, and then lysed in 1% NP40 in TSE (50 mM tris, 100 mM NaCl, 1 mM EDTA, pH 8.0) supplemented with 0.5 M NaF, 0.1M NaPPi, 0.5 M NaVO₄, 0.1 M PMSF, and complete protease inhibitors (Roche).

*Immunoprecipitation.* c-Fms is immunoprecipitated from FDC-c-fins cell extracts using 2.5µg/mL of anti-c-fms antibody (2-4A5, Santa Cruz Biotechnology Inc) and protein G Sepharose (Amersham). Immunoprecipitations are carried out for 2 h at 4°C, and samples are washed extensively and resuspended in 30 µL of reducing (anti-phosphotyrosine blots) or non-reducing (anti-c-fms blots) loading buffer. Equivalent amounts of protein as determined using a Micro BCA^{™} Protein Assay Reagent (Pierce) are used in each IP.

*Western Blot Analysis.* Immunoprecipitates are run on an 8% SDS-PAGE, and electroblotted to PVDF membrane (Amersham). Membranes are probed with anti-phosphotyrosine antibodies (mixture of 1/1000 PY20 (Santa Cruz Biotechnology Inc) and 1/2000 4G10 (Cell Signalling Technology^{®})) or an anti-c-fms antibody (R&D Systems). Detection is carried out using alkaline-phosphatase conjugated anti-mouse Ig antibody, and developed using ECF substrate (Amersham). The membrane is imaged using a Typhoon 9410 (Amersham) at 488nm excitation, and quantitation performed using ImageQuant^{™} software.

*Flow Cytometric Analysis of c-Fms Expression.* FDC-c-fins cells (5x10⁵) are cultured for 1h in serum-free IMDM in the presence of Compound I , then stained with 0.5 µg of anti-c-fms antibody. Bound antibody is detected by staining with an R-phycoerythrin conjugated anti-mouse antibody (SouthernBiotech), and cells analysed using a Coulter XL-MCS analytical flow cytometer.

*Statistical and Pharmacokinetic data Analysis.* Data is analysed using ANOVA, and differences are considered to be statistically significant when the probability value is <0.05. The calculation of IC50 values is performed using the Hill Equation, y=100/(1+10^{(logIC50-x)xHillSlope)}), where y is the level of inhibition and x is logarithmic drug concentration.

### Results

### Anti-c-Fnzs Inhibits Growth of Monocyte/Macrophage Colonies Stimulated with M-CSF or GM-CSF.

Compound I suppresses M-CSF or GM-CSF stimulated growth of cells of the monocyte/macrophage lineage isolated from normal donors, see e.g. Dewar, A.L et al., 2003 Leukemia 17:1713-21. In contrast to the related class III receptor tyrosine kinases c-kit and PDGFR, phosphorylation of c-fms has been reported to be unaffected by Compound I up to a concentration of 10 µM, see e.g. Buchdunger, E. et al., 2000. J Pharmacal Exp Ther 295:139-145.

Colony cultures established using bone marrow mononuclear cells from normal donors were stimulated with 4HGF or 5HGF, and the effect of anti-c-kit antibodies in combination with Compound I is examined on monocyte/macrophage growth, see Table 1A below. In the absence of Compound I, the addition of anti-c-kit to cultures stimulated with 4HGF had no effect on colony number. The dose of anti-c-kit (1µg/mL) used in these experiments is shown to be sufficient to completely block the SCF receptor, as its addition to cultures stimulated with 4HGF plus SCF (5HGF) reduced colony growth to the same level as cultures stimulated with 4HGF alone, see Table 1A below. The lack of an anti-c-kit antibody effect on monocyte/macrophage growth in the absence of added SCF and Compound I suggests that targeting of the c-kit signalling pathway following autocrine SCF production cannot account for the inhibition. In the following tables the abbreviation "SEM" stands for standard error of the mean and "cc" for concentration.

**Table 1A: Compound I inhibits monocyte/macrophage colony formation through inhibition of c-fms. MNC from normal BM are stimulated with 4HGF (IL-3, IL-6, G-CSF, GM-CSF), 4HGF + anti-c-kit antibodies (+KIT), 5HGF (IL-3, IL-6, G-CSF, GM-CSF, CSF), 5HGF + anti-c-kit antibodies (+KIT), and the effect on monocyte/macrophage colony formation examined.**

| Compound I Cc in micromol | 5HGF | 5HGF SEM | 4HGF | 4HGF SEM | 5HGF +KIT | 5HGF +KIT SEM | 4HGF +KIT | 4HGF +KIT SEM |
|---|---|---|---|---|---|---|---|---|
| 0 | 100 | 14.43 | 38.46 | 11.27 | 53.85 | 1.92 | 39.1 | 7.88 |
| 0.3 | 37.82 | 6.78 | 24.36 | 11.23 | 50.64 | 7.05 | 21.79 | 3.9 |
| 1 | 33.33 | 6.31 | 42.31 | 21.18 | 48.08 | 10.18 | 21.15 | 9.09 |
| 5 | 2.56 | 1.28 | 3.85 | 3.85 | 7.69 | 1.11 | 3.21 | 2.31 |
| 10 | 0.64 | 0.64 | 0 | 0 | 4.49 | 1.7 | 5.13 | 3.21 |
| 30 | 0 | 0 | 0 | 0 | 0 | 0 | 0 | 0 |

To examine the role of c-fms in colony growth, anti-c-fins antibodies are added to cultures following stimulation with M-CSF or GM-CSF. In M-CSF stimulated cultures, only monocyte/macrophage colonies are observed, which is inhibited by up to 80% in the presence of 1.0 µM Compound I, see Table 1B below.

**Table 1B: Growth of monocyte/macrophage from CD34+ progenitors stimulated with M-CSF is inhibited by the addition of anti-c-fms antibodies.**

| | Macrophage | SEM | Macrophage | SEM |
|---|---|---|---|---|
| anti-c-fms | - | - | + | + |
| M-CSF | + | + | + | + |
| Compound I Cc in µM | | | | |
| 0 | 100 | 12.95 | 0 | 0 |
| 1 | 15.57 | 3.895 | 0 | 0 |
| 5 | 0 | 0 | 0 | 0 |
| 10 | 0 | 0 | 0 | 0 |

Anti-c-fms antibody is sufficient to completely inhibit monocyte/macrophage colonies, demonstrating dependence of growth on M-CSF, see Table 1B above.

The addition of 1.0 µM Compound I to GM-CSF stimulated cultures reduced monocyte/macrophage colony growth by approximately 80%. In contrast, eosinophil growth is unaffected by Compound I at concentrations less than 10.0µM, see Table 1C below.

**Table 1C: Growth of monocyte/macrophage colonies from CD34+ progenitors stimulated with GM-CSF. Neither Compound I nor anti-c-fms antibodies affect eosinophil colony growth following stimulation with GM-CSF.**

| | Macrophage | SEM | Eosinophil | SEM | Macrophage | SEM | Eosinophil | SEM |
|---|---|---|---|---|---|---|---|---|
| anti-c-fms | - | - | - | - | + | + | + | + |
| GM-CSF | + | + | + | + | + | + | + | + |
| Compound I cc in µM | | | | | | | | |
| 0 | 100 | 7.14 | 100 | 7.36 | 0 | 0 | 95.8 | 10.20 |
| 1 | 28.86 | 5.79 | 94.15 | 6.50 | 0 | 0 | 93.96 | 11.67 |
| 5 | 0 | 0 | 83.47 | 8.35 | 0 | 0 | 66.89 | 12.45 |
| 10 | 0.53 | 0.53 | 59.62 | 9.52 | 0 | 0 | 24.35 | 6.84 |

The addition of an anti-c-fms antibody to GM-CSF stimulates cultures completely abrogated monocyte/macrophage colony growth while eosinophil growth is unaffected, suggesting that while GM-CSF directly stimulates eosinophil growth, it indirectly stimulates the growth of monocyte/macrophage colonies.

GM-CSF stimulation of monocytes induces M-CSF protein secretion and since GM-CSF indirectly stimulates anti-c-fms inhibitable monocyte/macrophage colony growth, it is examined if GM-CSF induces autocrine production of M-CSF in culture system. Low levels (20 pg/mL) of M-CSF are detected 24 h after cultures are established (data not shown) with these levels increasing steadily over the 5 days to approximately 70 pg/mL of M-CSF. The addition of 1.0 µM Compound I has no effect on M-CSF production by the cultured monocytes, while a 30% decrease in M-CSF production at day 5 is seen at 5.0 µM Compound 1. The maximum level of M-CSF produced is estimated to be 70pg/mL, which is 300-500 fold lower than the concentration of added M-CSF used in this study. It is possible that a suboptimal concentration of M-CSF is sufficient to induce the growth and differentiation of monocytes, with GM-CSF incapable of supporting monocyte/macrophage growth alone, but acting synergistically to potentiate the effect of M-CSF.

*Compound I Inhibits the Proliferation of an M-CSF Dependent Cell Line.* Since Compound I appears to be mediating its inhibitory effect on monocyte/macrophage development through c-fms, the effect of Compound I on a cell line that is dependent on either murine IL-3 or human M-CSF is investigated. Control cultures stimulated with murine IL-3 showed no Compound I-specific effects on cell growth at 12 or 24 h across the range of Compound I doses examined, e.g. see Table 2A. At 48 h, FDC-c-fms proliferation in the presence of IL-3 is reduced by 15% at 2.5 µM Compound I and 40% at 5.0 µM Compound I, suggesting that Compound I has a mildly toxic effect on these cells.

**Table 2A**

| | | | | | | |
|---|---|---|---|---|---|---|
| IL-3 | | | | | | |
| Compound I cc in µM | 12 H | SEM | 24 H | SEM | 48 H | SEM |
| 0 | 125433 | 2899 | 257726 | 6837 | 1123171 | 25940 |
| 0.5 | 131724 | 1659 | 281975 | 5626 | 1107666 | 29249 |
| 1 | 126472 | 3478 | 264007 | 11736 | 1068150 | 69927 |
| 2.5 | 131857 | 3210 | 247613 | 8415 | 940233 | 34057 |
| 5 | 106917 | 3304 | 197435 | 6599 | 682771 | 28252 |

**Table 2B**

| | | | | | | |
|---|---|---|---|---|---|---|
| M-CSF | | | | | | |
| Compound I cc in µM | 12 H | SEM | 24 H | SEM | 48 H | SEM |
| 0 | 132174 | 5401 | 310859 | 10028 | 1453642 | 50607 |
| 0.5 | 133296 | 3123 | 339185 | 5220 | 1577777 | 108291 |
| 1 | 130010 | 3034 | 311035 | 9293 | 1362802 | 63713 |
| 2.5 | 117025 | 3559 | 174818 | 15978 | 295134 | 7763 |
| 5 | 68324 | 2040 | 20830 | 1708 | 20149 | 4695 |

**Tables 2A and 2B:** Compound I inhibits M-CSF but not IL-3 stimulated growth of a c-fms expressing cell line at therapeutic concentrations. Cell counts (cells/mL) are performed at 12, 24 and 48 h time points. Control cultures stimulated with murine IL-3 showed minor growth inhibition at 48 h at concentration of Compound I of 2.5 µM or greater (2A). No effect is seen at 12 and 24 h time points. An IC₅₀ value of 5.9 µM is predicted using the sigmoidal model indicating minor drug toxicity at higher concentrations of Compound I (not shown). Stimulation of cells with M-CSF demonstrates growth inhibition at 5.0 µM Compound I at 12 h and 2.5 µM at 24 and 48 h (2B) with an IC₅₀ value of 1.1 µM Compound I.

Where M-CSF is the sole source of stimulation, a 50% inhibition of cell growth is seen at 5.0µM Compound I 12h after the initiation of the culture. At 24 h, cell counts are up to 45% lower at 2.5 µM Compound I than cultures not stimulated with Compound I, and at 5.0 µM Compound I, cell counts are lower than seeded values. The effect of Compound I on M-CSF stimulated FDC-c-fms cultures is most profound at 48 h, where 2.5 µM Compound I reduced cell counts by 80% relative to controls, and at 5.0 µM Compound I, the concentration of cells is lower than the seeded level.

*Compound I Inhibits the Phosphorylation of c-Fms.* To determine if Compound I mediated an inhibitory effect on the M-CSF receptor directly, the effect of Compound I on the phosphorylation c-fins is examined on FDC-c-fins cell lines. Starved FDC-c-fms cells that are not stimulated with M-CSF displayed no c-fms phosphorylation. Starved FDC-c-fins cells that are stimulated with M-CSF exhibited receptor phosphorylation, and 1.0 µM Compound I reduces this phosphorylation by approximately 30%. At 2.5 µM Compound I, c-fms phosphorylation is reduced by 75%, and at 5.0 µM Compound I, no significant phosphorylation is observed. Analysis of the data yielded an IC₅₀ value for Compound I inhibition of c-fms phosphorylation of 1.42 µM, similar to the value obtained in the proliferation experiments.

*Compound I does not affect c-Fms Protein Expression.* c-Fms is expressed at low levels on monocytes, and its expression markedly increases during differentiation to macrophages. In the absence of M-CSF, the mature cell-surface form of c-fms is relatively stable, however ligand binding down-regulates receptor expression by internalisation and degradation within lysosomes. Since phosphorylation of the M-CSF receptor is inhibited by treatment with Compound I, Western blots are probed for c-fms protein to confirm this is not due to a decrease in c-fms expression. Two c-fms bands are detected in these blots, with the 170kDa band representing the fully glycosylated c-fms protein, and the 130kDa band representing the immature, non-glycosylated form. The intensity of both the 170kDa and 130kDa bands is quantitated, and while much higher levels of the 170kDa protein are consistently detected, the expression of both forms of c-fms are unaffected by Compound I treatment, see Table 4A below. The lack of effect of Compound I on the expression of c-fms is confirmed using flow cytometry, with no difference in surface expression of c-fms in FDC-c-fms cells cultured in 0.5µM-2.5 µM Compound I, and marginally lower expression at 5.0 µM Compound I, see Table 4B.

**Table 4A: Western blot quantitation of c-fms species. IC = isotype control.**

| | c-fms species | |
|---|---|---|
| | 130kd | 170kd |
| no M-CSF | 152478 | 530176 |
| M-CSF + Compound I cc in µM 0 | 188656 | 537484 |
| 0.5 | 146299 | 455754 |
| 1 | 147714 | 460596 |
| 2.5 | 117604 | 438912 |
| 5 | 97043 | 454761 |
| ControlIC | 19097 | 153239 |

**Table 4B: Surface c-fms detected by flow cytometry.**

| Compound I cc in µM | Mean Fluorescence intensity |
|---|---|
| 0 | 63 |
| 0.5 | 69 |
| 1 | 52 |
| 2.5 | 54 |
| 5 | 37 |

It is demonstrated that the *in vitro* profile of the protein-tyrosine kinase inhibitor Compound I can be extended to include c-fms. The potency of inhibition is lower than that observed for Abl (IC₅₀ = 0.025 µM), c-kit (IC₅₀ = 0.1 µM) or PDGF (IC₅₀ = 0.25 µM) receptor tyrosine kinases. Western blotting demonstrates a Compound I concentration of 1.4 µM required to inhibit c-fms tyrosine phosphorylation by 50%. In this present study, the effect of Compound I on the phosphorylation of c-fins is examined on c-fms immunoprecipitates following specific receptor stimulation with saturating doses of M-CSF.

Compound I can be used in the treatment of diseases involving abnormal c-fms activation, including common cancers such as breast cancer and epithelial ovarian cancer, and inflammatory conditions such as rheumatoid arthritis. Abnormal expression of c-fms has been demonstrated on a range of human cancers including carcinomas of the breast and ovary, and activation of c-fms has been demonstrated to stimulate tumour invasion by a urokinase-dependent mechanism, see Kacinski B.M. 1997, Mol Reprod Dev 46:71-4, Sapi E and B.M. Kacinski, 1999, Proc Soc Exp Biol Med 220:1-8. Abnormal expression of c-fins in breast tumours and advanced epithelial ovarian carcinomas correlates with tumour cell invasiveness and adverse clinical prognosis, and M-CSF produced by breast tumours has been implicated in the promotion of bone metastasis in breast cancer, see Toy E.P. et al., 2001, Gynecol. Oncol. 80:194-200, Sapi E. 2004 Exp Biol Med 229:1-11. The potent inhibitory effect of Compound I on c-fins phosphorylation also has important implications with regard to potential drug toxicity. Outside the haemopoietic system, c-fms signalling plays an important role in pregnancy, affecting pre-implantation embryo development and mammary gland development, see Pollard J.W. 1997, Mol Reprod Dev 46:54-60. Through M-CSF stimulation, c-fins also plays an important role in bone metabolism and inflammatory processes, Fixe P. and V. Praloran, 1998, Cytokine 10: 32-37. While evidence to date demonstrates that Compound I is well tolerated by patients, potential effects of Compound I on these processes must be considered as a consequence of long term Compound I treatment.

### Example 2: Capsules with 4-[(4-methyl-1-piperazin-1-ylmethyl)-N-[4-methyl-3-[[4-(3-pyridinyl)-2-pyrimidinyl]amino]phenyl]benzamide monomethanesulfonate, β-crystal form

Capsules containing 119.5 mg of the compound named in the title (= SALTI) corresponding to 100 mg of COMPOUND I (free base) as active substance are prepared in the following composition:

| | | |
|---|---|---|
| Composition | SALT I | 119.5 mg |
| | Avicel | 200 mg |
| | PVPPXL | 15 mg |
| | Aerosil | 2 mg |
| | Magnesium stearate | 1.5 mg |
| | | 338.0 mg |

The capsules are prepared by mixing the components and filling the mixture into hard gelatin capsules, size 1.

## Claims

1. Use of 4-(4-methylpiperazin-1-ylmethyl)-N-[4-methyl-3-(4-(pyridin-3-yl)pyrimidin-2-ylamino)phenyl]-benzamide (Compound I) for the manufacture of a medicament for the treatment of c-fms-associated diseases, wherein the c-fms-associated disease is selected from the group consisting of choriocarcinoma, embryonal carcinoma, brain microglial tumors, sarcoidosis, microglial cell involvement in normal and variant Creutzfeld-Jacob disease, or amyotrophic lateral sclerosis.

2. The use according to claim 1 wherein Compound I is in the monomethane sulfonate salt form.

## Patentansprüche

1. Verwendung von 4-(4-Methylpiperazin-1-ylmethyl)-N-[4-methyl-3-(4-(pyridin-3-yl)pyrimidin-2-ylamino)phenyl]-benzamid (Verbindung I) zur Herstellung eines Arzneimittels zur Behandlung von c-fms-assoziierten Erkrankungen, wobei die c-fms-assoziierte Erkrankung ausgewählt ist aus der Gruppe, bestehend aus Choriokarzinom, embryonalem Karzinom, mikroglialen Gehirntumoren, Sarkoidose, mikroglialer Zellbeteiligung an normaler sowie an Varianten der Creutzfeld-Jacob-Krankheit, oder amyotrophe Lateralsklerose.

2. Die Verwendung gemäß Anspruch 1, wobei Verbindung I in Form des Monomethansulfonatsalzes vorliegt.

## Revendications

1. Utilisation du 4-(4-méthylpipérazin-1-ylméthyl)-N-[4-méthyl-3-(4-(pyridin-3-yl)pyrimidin-2-ylamino)phényl]benzamide (Composé I) pour la fabrication d'un médicament destiné au traitement des maladies associées au c-fms, la maladie associée au c-fms étant choisie dans le groupe constitué par le chorio-carcinome, le carcinome embryonnaire, les tumeurs cérébrales de la microglie, la sarcoïdose, l'implication des cellules de la microglie dans la maladie de Creutzfeld-Jacob normale ou sa variante, ou la sclérose latérale amyotrophique.

2. Utilisation selon la revendication 1, où le Composé I est sous forme de sel sulfonate de monométhane.
